# EUROPEAN PATENT SPECIFICATION

(11) **EP 2 526 978 B1**
(45) Date of publication and mention of the grant of the patent: **09.12.2020**
(21) Application number: 12164605.3
(22) Date of filing: 18.04.2012
(51) Int. Cl.: A61L 27/50, C12M 3/00

(54) **Device and method of generating in vitro blood vessels**
Vorrichtung und Verfahren zur Erzeugung von In-vitro-Blutgefäßen
Dispositif et procédé de génération de vaisseaux sanguins in vitro

(30) Priority: 18.04.2011 US 201113089130
(43) Date of publication of application: 28.11.2012
(73) Proprietor: SNU R&DB Foundation, Seoul 151-742 (KR)
(72) Inventor: Jeon, Noo Li, Seoul 151-744 (KR); Kim, Sudong, Seoul 151-846 (KR); Lee, Hyunjae, Gyeonggi-do 463-732 (KR); Chung, Minhwan, Seoul 135-082 (KR)
(74) Representative: Schiweck Weinzierl Koch Patentanwälte Partnerschaft mbB

(56) References cited:
- EP-A1- 2 302 353
- US-A1- 2008 261 306
- CARLOS P. HUANG ET AL.: "Engineering microscale cellular niches for three-dimensional multicellular cocultures", LAB CHIP, vol. 9, 2009, pages 1740-1748, XP55006400,
- WEI TAN ET AL.: "Microscale multilayer cocultures for biomimetic blood vessels", JOURNAL OF BIOMEDICAL MATERIALS RESEARCH PART A, vol. 72A, no. 2, 1 February 2005 (2005-02-01), pages 146-160, XP002685045,

## Description

### TECHNICAL FIELD

Provided herein are devices and methods for generating in vitro blood vessels.

### BACKGROUND ART

A circulatory system is constructed from a network of vessels, which serves a wide range of functions from cellular and biochemical transport, nutrient and oxygen exchange to temperature regulation while maintaining a degree of plasticity throughout the development and life of an organism. Development, function and homeostasis of blood vessels are major components of diverse physiological and pathological phenomena. The foundation of such efficient system is based on the primary building block, the vascular endothelial cells (EC), which regulates transvascular transport, vasodilation, and vessel formation and regression. As with any system of such complexity, malfunction and dysregulation can lead to a multitude of pathologies. Therefore, elucidating the cellular and molecular mechanisms underlying blood vessel formation is essential in developing new therapeutic interventions.

Numerous *in vivo* and *in vitro* experimental systems have been developed to investigate vessel dynamics and function. ECs cultured on 2D substrates are widely used for the investigation of barrier function, mechanosensitive response of endothelium, and transendothelial migration of blood-borne cells including leukocytes and circulating tumor cells (CTCs). However, such 2D endothelial monolayers lack three-dimensional context that provides signals to induce cells to resemble their innate characteristics. Furthermore, no single approach has successfully demonstrated the reconstitution of complex vascular physiology by integrating natural 3D morphogenesis as well as microcirculation through the perfused vascular network. In this regard Carlos et al. describes engineering microscale cellular niches for three-dimensional multicellular cocultures (Carlos et al. (2009) "Engineering microscale cellular niches for three-dimensional multicellular cocultures" LAB CHIP, vol. 9, pages 1740-1748). Furthermore, Tan and Desai disclose microscale multilayer cocultures for biomimetic blood vessels (Tan and Desai (2005) "Microscale multilayer cocultures for biomimetic blood vessels" Journal of Biomedical Materials Research, vol. 72A, no. 2, pages 146-160). US 2008/261306 A1 describes a method for creating networks of perfusable microvessels *in vitro.* An *in vitro* model system that can capture detailed dynamics of ECs during vessel formation and integrate essential physiological components contributing functional characteristics of *in vivo* vessels would provide a useful experimental platform for vascular biology research. Furthermore, as discrepancies between human and animal physiology, compromised predictive potential of current *in vitro* systems lead to the failure of clinical trials during new drug development, novel *in vitro* model which reflects complex vascular physiology would benefit pharmaceutics by providing effective and accurate evaluation of anti-angiogenic or vasoactive compounds.

### DISCLOSURE OF INVENTION

### TECHNICAL PROBLEM

The present application relates, in general, to the field of devices and methods for generating perfusable, connected network of blood vessels with lumens *in vitro* in 3D gels.

### SOLUTION TO PROBLEM

In one exemplary embodiment of the present invention, the blood vessel-generating device comprising a) a sink channel in fluid communication with a sink inlet; b) a source channel in fluid communication with a source inlet substantially parallel to the sink channel; c) a blood vessel-forming channel in fluid communication with a blood vessel-forming channel inlet, disposed in contact with the sides of the sink channel and the source channel between the sink channel and the source channel, substantially parallel to the source channel and the sink channel; d) a first culture channel in fluid communication with a first culture channel inlet, disposed in contact with the other side of the sink channel, substantially parallel to the sink channel; and e) a second culture channel in fluid communication with a second culture channel inlet, disposed in contact with the other side of the source channel, substantially parallel to the source channel, wherein a plurality of microstructures configured to allow interaction between biochemical materials included in each channel are arranged in the interfaces of the respective adjacent channels at set intervals, and wherein the sink channel is disposed between the blood vessel-forming channel and the first culture channel, and the source channel is disposed between the blood vessel-forming channel and the second culture channel.

Another exemplary embodiment of the present invention relates to a method of generating a blood vessel comprising a) injecting an ECM (extracellular matrix) and a blood vessel-forming cell into the blood vessel-forming channel of the blood vessel-generating device; b) injecting i) the ECM or ii) the ECM and a co-culturing cell into the first culture channel and/or the second culture channel of the blood vessel-generating device; and c) injecting an angiogenesis factor and/or a cell culture medium into the sink channel and/or the source channel of the blood vessel-generating device, and culturing the blood vessel-forming cell and the co-culturing cell; and
d) interacting the blood vessel-forming cell with the co-culturing cell via paracrine signaling.

The details of one or more embodiments of the disclosure are set forth in the accompanying drawings and the description below. Other features, objects, and advantages will be apparent from the description and drawings, and from the claims.

### ADVANTAGEOUS EFFECTS OF INVENTION

Devices and methods of 3-dimensionally generating *in vitro* blood vessels are provided.

### BRIEF DESCRIPTION OF THE DRAWINGS

FIG. 1 depicts an embodiment of the blood vessel-generating device.
FIG. 2 is a schematic diagram showing a mechanism of filling an ECM into a blood vessel-forming channel of the blood vessel-generating device according to the embodiment.
FIG. 3 is a schematic diagram showing some of the methods of generating a blood vessel using the blood vessel-generating device according to the embodiment.
FIG. 4 is a schematic diagram showing an experimental setup and cell seeding configuration to model vasculogenesis process using the device and method for generating a blood vessel according to the present invention.
FIG. 5 depicts a microscopic image of a blood vessel network generated by simulating a vasculogenesis process using the device and method for generating a blood vessel according to the present invention.
FIG. 6 is a schematic diagram showing an experimental setup and cell seeding configuration to model angiogenesis process using the device and method for generating a blood vessel according to the present invention.
FIG. 7 depicts a microscopic image of angiogenic sprouts and another blood vessel network generated by simulating an angiogenesis process using the device and method for generating a blood vessel according to the present invention.
FIG. 8 depicts another embodiment of a blood vessel-generating device.
FIG. 9 depicts a fluorescent microscopic image that shows FITC (Fluorescein Isothiocyanate) solution, with the molecular mass of 70 kDa, and fluorescent particles, whose radii are as long as 7 micrometers, introduced into the blood vessel network that is perfused and directly accessible through the source channel and the sink channel.
FIG. 10 depicts a fluorescent image that describes the upregulation of ICAM-1 expression on the apical side of the blood vessels by introducing cytokines that mediate inflammatory responses, including TNF-a and IL-1a, into the perfusable networks of blood vessels.
FIG. 11 depicts a fluorescent image which illustrates the cancer cells that were introduced into the blood vessel networks, simulating cancer cells disseminated from the original site into the blood stream that adhere and extravasate into the tissue of secondary metastatic sites.

### MODE FOR THE INVENTION

Devices and methods of 3-dimensionally generating in vitro blood vessels are provided.
The present invention provides a blood vessel-generating device comprising a) a sink channel in fluid communication with a sink inlet; b) a source channel in fluid communication with a source inlet substantially parallel to the sink channel; c) a blood vessel-forming channel in fluid communication with a blood vessel-forming channel inlet, disposed in contact with the sides of the sink channel and the source channel between the sink channel and the source channel, substantially parallel to the source channel and the sink channel; d) a first culture channel in fluid communication with a first culture channel inlet, disposed in contact with the other side of the sink channel, substantially parallel to the sink channel; and e) a second culture channel in fluid communication with a second culture channel inlet, disposed in contact with the other side of the source channel, substantially parallel to the source channel, wherein a plurality of microstructures configured to allow interaction between biochemical materials included in each channel are arranged in the interfaces of the respective adjacent channels at set intervals and
wherein the sink channel is disposed between the blood vessel-forming channel and the first culture channel, and the source channel is disposed between the blood vessel-forming channel and the second culture channel.

By the present method, an angiogenesis process can be simulated.

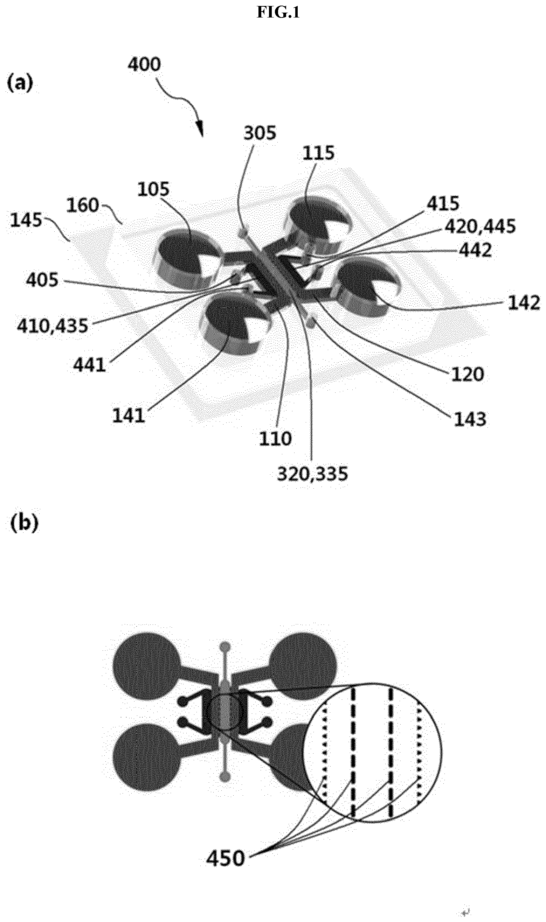

The embodiment of the blood vessel-generating device is shown in FIG. 1. The blood vessel-generating device 400 includes abase 145 associated with a substrate 160.

The substrate 160 includes a sink inlet 105 in communication with the sink channel 110, capable of injecting fluid into the sink channel 110, and a source inlet 115 in communication with a source channel 120, capable of injecting fluid into the source channel 120. Also, the substrate 160 includes a sink outlet 141 in communication with the sink channel 110, capable of discharging the injected fluid out of the sink channel 110, and a source outlet 142 in communication with the source channel 120, capable of discharging the injected fluid out of the source channel 120. FIG. 1 is a conceptual diagram showing the sink channel 110 and the source channel 120 including an inlet and an outlet. In another embodiment, the sink channel 110 and source channel 120 may form one channel in order to communicate with each other. In this case, the sink inlet 105 and the source inlet 115 may form one inlet, and the sink outlet 141 and the source outlet 142 may form one outlet. Also, it is possible to inject and discharge fluid through the sink inlet 105 and the source inlet 115 without using separate sink and sourceoutlets141 and 142.

Furthermore, the substrate 160 includes a blood vessel-forming channel 320 disposed in contact with the sides of the sink channel 110 and the source channel 120 between the sink channel 110 and the source channel 120, substantially parallel to the sink channel 110 and the source channel 120. Also, the substrate 160 includes a first culture channel 410 disposed in contact with the other side of the sink channel 110, substantially parallel to the sink channel 110 and a second culture channel 420 disposed in contact with the other side of the source channel 120, substantially parallel to the source channel 120. FIG. 1 is a conceptual diagram showing that each of the blood vessel-forming channel 320 and the first and second culture channels 410 and 420 includes inlets 305, 405 and 415 and outlets 143, 441 and 442. However, it is possible to inject and discharge the fluid through the inlets without using separate outlets.

The adjacent interfaces of the sink channel 110, the source channel 120, the blood vessel-forming channel 320, the first culture channel 410 and the second culture channel 420 are divided by arranging a plurality of microstructures 450 at set intervals so that biochemical materials in each channel can interact with each other. The microstructures 450 have fine column shapes with a length and height ranging from several tens to several hundreds of micrometers (µm). The plurality of microstructures 450 may be arranged at a distance of 500 µm or less. The distance may be suitably determined according to the purpose and conditions of an experiment.

A blood vessel-generating region 335 is 3-dimensionally formed by filling i) an ECM or ii) an ECM and a blood vessel-forming cell into the blood vessel-forming channel 320. The blood vessel-generating region 335 provides a space in which blood vessel-forming cells are 3-dimensionally proliferated and differentiated to form blood vessels. Since the plurality of microstructures 450 are arranged in the adjacent interface between the blood vessel-forming channel 320 and the sink channel 110 and the source channel 120 at set intervals, when i) an ECM or ii) an ECM and a blood vessel-forming cell are injected into the blood vessel-forming channel 320, i) the ECM or ii) the ECM and the blood vessel-forming cell form the blood vessel-generating region 325 in the blood vessel-forming channel 320 without any leakage of i) the ECM or ii) the ECM and the blood vessel-forming cell between the microstructures 450 due to the surface tension of i) the ECM or ii) the ECM and the blood vessel-forming cell. Meanwhile, since various biochemical materials in each channel may flow and diffuse through gaps between the plurality of microstructures 450, various angiogenesis factors and/or nutrient ingredients included in the sink channel 110 and the source channel 120 may be supplied to the blood vessel-forming channel 320. Also, blood vessels generated in the blood vessel-generating region 335 extend through the gaps formed between the plurality of microstructures 450 to be in communication with the sink channel 110 and the source channel 120, thereby forming an inlet/outlet of the blood vessel and making the blood vessel possible to be perfused with the fluid included in the source channel 120 or the sink channel 110. Therefore, the reaction of the blood vessels may be observed in real time and recorded when directly transferring various biochemical and biophysical materials and signals into the blood vessels through the sink channel 110 and the source channel 120 which are in communication with the gaps between the microstructures 450. Therefore, the number of inlets and outlets of a blood vessel network to be generated may be adjusted by changing the shape, length and number of the microstructures disposed between the blood vessel-forming channel 320 and the sink channel 110 and the source channel 120. It will be understood that various parameters such as the shape of the microstructures may be made without departing from the scope of embodiments in Fig 1. Accordingly, other parameters may be changed within the scope of the purpose and configuration of the experiment.

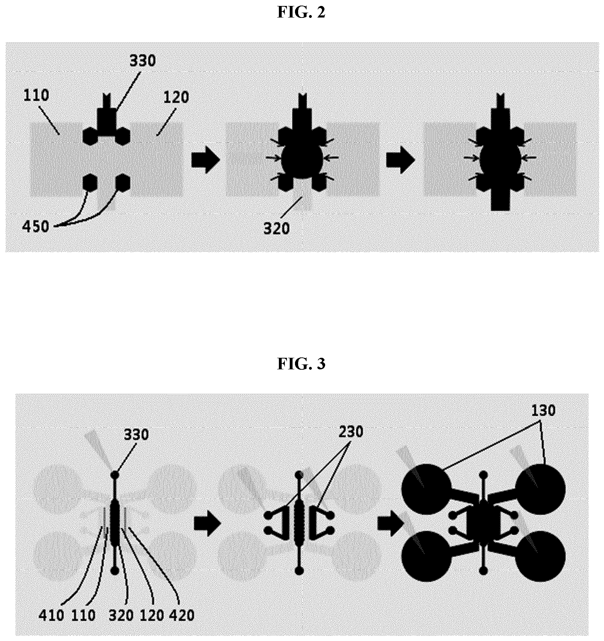

FIG. 2 is a schematic diagram showing a mechanism of filling i) an ECM or ii) an ECM and a blood vessel-forming cell into a blood vessel-forming channel. According to the present invention, the substrate 160 may be made of a hydrophobic material. i) An ECM or ii) an ECM and a blood vessel-forming cell 330 injected into the blood vessel-forming channel 320 forms the menisci between the plurality of microstructures 450. In this case, when the pressure inside the liquid menisci are put under a certain level of a pressure (a threshold pressure level), the menisci do not proceed into the sides of the sink channel 110 and the source channel 120 due to the resisting surface tension. A range of the pressure in which the menisci may stop between the microstructures without allowing the menisci to proceed into the sides of the sink channel 110 and the source channel 120 is affected by the gaps between the microstructures and the heights of the microstructures. Therefore, these two parameters (the gaps between the microstructures and the heights of the microstructures) may be optimized to adjust a threshold pressure level. The specific method of adjusting the threshold pressure level is found in Carlos P. Huang et al. (Engineering microscale cellular niches for three-dimensional multicellular cocultures, Lab Chip, 2009, 9, 1740-1748). When i) an ECM or ii) an ECM and a blood vessel-forming cell are injected into a blood vessel-forming channel, the menisci may be effectively trapped between microstructures by adjusting the gaps between the microstructures and the heights of the microstructures, thereby precisely adjusting the filling of i) the ECM or ii) the ECM and the blood vessel-forming cell between channels that are not completely physically separated from each other. This is also applied in the same manner even when i) an ECM or ii) an ECM and a co-culturing cell are filled into the first and second culture channels.

Cell culture regions 435 and 445 are 3-dimensionally formed by filling i) an ECM or ii) an ECM and a co-culturing cell into the first and second culture channels 410 and 420. Since the plurality of microstructures 450 are arranged in the adjacent interfaces between the first and second culture channels 410 and 420 and the sink channel 110 and the source channel 120 at set intervals, when i) an ECM or ii) an ECM and a co-culturing cell are injected into the first and second culture channels 410 and 420, i) the ECM or ii) the ECM and the co-culturing cell form cell culture regions 435 and 445 in the first and second culture channels 410 and 420 without any leakage of i) the ECM or ii)the ECM and the co-culturing cell between the microstructures 450 due to the surface tension of i) the ECM or ii) the ECM and the co-culturing cell. The cell culture regions 435 and 445 provide a space in which co-culturing cells may be 3-dimensionally cultured. This 3-dimensionalmulticellular co-culturing may simulate an *in vivo* environment more physiologically, and thus it is possible to promote production and secretion of various signaling materials from the co-culturing cells. Meanwhile, since the various signaling materials produced in the first and second culture channels 410 and 420 and various biochemical material such as an angiogenesis factor included in the sink channel 110 and the source channel 120 may diffuse through the gaps between the plurality of microstructures 450, it is possible to perform the active interaction of biochemical materials between the first culture channel 410 and the sink channel 110 and between the second culture channel 420 and the source channel 120.

The sink channel 110 and the source channel 120 provide a passage through which the flow of fluid is allowed. Here, the fluid may include a cell culture medium, a variety of angiogenesis factors, etc. As each of the sink channel 110 and the source channel 120 is disposed between the blood vessel-forming channel 335 and the first and second culture channels 410 and 420, cell culture media and/or angiogenesis factors are supplied to the blood vessel-forming channel 335 and the first and second culture channels 410 and 420. In this manner, the sink channel 110 and the source channel 120 provide a passage through which two cell groups (a blood vessel-forming cell and a co-culturing cell) in the blood vessel-forming channel 335 and the first and second culture channels 410 and 420 may interact with each other via paracrine signaling, and the blood vessel-forming cells and the co-culturing cells may be cultured for an extended period of time. The paracrine interactions between the blood vessel forming cells and the co culturing cells are known to promote the morphogenesis of blood vessel forming cells into the network of blood vessels. Also, since the blood vessel-forming cells and the co-culturing cells are physically separated from each other by the sink channel 110 and the source channel 120, the blood vessel-forming cells and the co-culturing cells may be readily observed and analyzed independently from each other. In particular, since the sink channel 110 and the source channel 120 are in communication with the inlets/outlets of vascular networks formed at gaps between the microstructures 450, it is possible to exert a variety of biochemical and biophysical materials and signals into the blood vessels through the gaps. Therefore, it is possible to simulate the interaction between the variety of biochemical and biophysical signals and the blood vessels, thereby imaging and quantifying the response of the blood vessels to various stimuli in real time.

When the blood vessel-generating device 400 according to the present invention is used to generate a blood vessel, the kinds and characteristics of an ECM, a blood vessel-forming cell, a cell culture medium and a co-culturing cell are the same as described below. In one specific embodiment of the present invention, the ECM may be, for example, at least one selected from a collagen gel, a fibrin gel, Matrigel, a self-assembled peptide gel, a polyethylene glycol gel and an alginate gel. A fibrin gel is used in this embodiment. The ECM may contain at least one selected from the group consisting of a drug compound, a soluble factor, an insoluble factor, a biomolecule, a protein, a nanomaterial and siRNA to quantify and evaluate the effects and efficacy of them on blood vessel formation and function or to estimate the pro- or anti-angiogenic characteristics of them as therapeutic agents.

In another specific embodiment of the present invention, the blood vessel-forming cell may be, for example, at least one selected from an endothelial cell, an epithelial cell, a cancer cell, a stem cell, a stem cell-derived cell and a endothelial progenitor cell. The blood vessel-forming cell may be understood to include mutated cells, genetically modified and transfected cells thereof. A human umbilical vain endothelial cell (HUVEC) is used in this embodiment. In the specification, the term "blood vessel-forming cell" refers to a cell that forms a blood vessel by interaction of angiogenesis-inducing agents included in a cell culture medium or angiogenesis factors produced from a co-culturing cell. The blood vessel-forming cell may form a blood vessel through vasculogenesis or angiogenesis that closely mimics the blood vessel forming process observed *in vivo.* For example, blood vessel endothelial cells, such as HUVEC, a human microvascular endothelial cell, a human brain microvascular endothelial cell, and a human lymphatic endothelial cell separated from various human organs may be used as the blood vessel-forming cell. Not only human-derived cell lines, diverse cell lines separated from mouse, rat, cattle, pig may be used as the blood vessel-forming cell. Also, a cancer cell may be used to conduct research on cancer growth and metastasis mechanisms. As described above, the kind of the blood vessel-forming cell may be suitably selected by those skilled in the art according to the purpose of the experiments.

In still another specific embodiment of the present invention, any one of cell culture mediums known in the art may be used as the cell culture medium. An EGM-2 medium (LONZA) is used in this embodiment. The cell culture medium may contain at least one selected from the group consisting of a drug compound, a soluble factor, an insoluble factor, a biomolecule, a protein, a nanomaterial and siRNA to quantify and evaluate the effects and efficacy of them on blood vessel formation and function or to estimate the pro- or anti-angiogenic characteristics of them as therapeutic agents.

In yet another specific embodiment of the present invention, the co-culturing cell may be a cell that secretes a biochemical material required to form a blood vessel, such as an angiogenesis-inducing agent, through the interaction with the blood vessel-forming cell. The co-culturing cell may be, for example, at least one selected from an astrocyte, a glial cell, a mesothelial cell, a fibroblast, a smooth muscle cell, a cancer cell, a pericyte, a neuroglial cell, a stem cell, a stem-cell derived cell and a cell to interact with endothelium.

The co-culturing cell may be understood to include mutated cells, genetically modified, and transfected cells thereof. When a blood vessel to be generated is a blood vessel in the brain, the co-culturing cell would be preferably an astrocyte, a glial cell, a mesothelial cell or a fibroblast. When a blood vessel to be generated is a blood vessel other than the blood vessel in the brain, the co-culturing cell would be preferably a fibroblast or a smooth muscle cell. Also, in order to conduct research on the relationship between cancer and angiogenesis, a cancer cell may be used as the co-culturing cell. A human lung fibroblast is used in this embodiment. The co-culturing cell is the human lung fibroblast and HUVEC in this embodiment. Accordingly, the co-culturing cell type, combination and cell seeding configuration of the co-culturing thereof are within the scope of the purpose and configuration of the experiment.

The addition of the angiogenesis factor, the cell culture medium and the co-culturing cell may be performed to provide a suitable environment for the growth, proliferation and morphogenesis of the blood vessel-forming cell. The kinds and compositions of the angiogenesis factor, the cell culture medium and the co-culturing cell may be suitably selected by those skilled in the art.

The present invention also provides a method of generating a blood vessel comprising a) injecting an ECM and a blood vessel-forming cell into the blood vessel-forming channel of the blood vessel-generating device; b) injecting i) the ECM or ii) the ECM and a co-culturing cell into the first culture channel and/or the second culture channel of the blood vessel-generating device; and c) injecting an angiogenesis factor and/or a cell culture medium into the sink channel and/or the source channel of the blood vessel-generating device and culturing the blood vessel-forming cell and the co-culturing cell; and d) interacting the blood vessel-forming cell with the co-culturing cell via paracrine signaling. By the present method, vasculogenesis process can be simulated.

FIG. 3 depicts a method of generating a blood vessel using the blood vessel-generating device 400 according to the present invention. Here, the method of generating a blood vessel includes injecting an ECM and a blood vessel-forming cell 330 into the blood vessel-forming channel 320, injecting i) the ECM or ii) the ECM and a co-culturing cell 230 into the first culture channel 410 and second culture channels 420, and injecting a fluid 130 including a cell culture medium and/or various angiogenesis factors into the sink channel 110 and the source channel 120. When the ECM, the cells, the cell culture media and the various angiogenesis factors are injected into each channel, additional external experimental equipment is not required, and they may be simply injected by pipetting. Also, the blood vessel-forming cells and the co-culturing cells may be mixed with an ECM to be injected into the blood vessel-forming channel or the first culture channel and the second culture channel, and may also be injected through the sink channel or the source channel to make them adhere on the side wall of ECM formed between the microstructures depending on purpose of the experiment. The method using the blood vessel-forming cells mixed with ECM and injected into the blood vessel-forming channel may mimic vasculogenic formation of vessel networks, and the method using the blood vessel-forming cells attached on ECM may mimic angiogenesis process allowing formation of angiogenic sprouts and tip cells.

FIG. 3 is a conceptual diagram showing the one possible procedure of serially injecting and patterning i) an ECM or ii) an ECM and a blood vessel forming/co culturing cells into the each channel, which is followed by loading of cell culture media or angiogenic factors into the source and the sink channel. i) An ECM or an ii) ECM and a blood vessel-forming cell 330 are injected into the blood vessel-forming channel 320, and then i) the ECM or ii) the ECM and a co-culturing cell 230 are injected into the first and second culture channels 410 and 420. However, the injection order does not affect the formation of a blood vessel. Therefore, the order of injecting the materials such as an ECM or a co-culturing cell into each channel may be varied according to the experimenter's convenience and characteristics of experiments. The ECM or the co-culturing cell injected into each channel may be polymerized by increased temperature, chemical reaction or light irradiation, depending on the kind of the ECM or the co-culturing cell. After this polymerizing process, a nutrient ingredient and an angiogenesis factor may be supplied to the blood vessel-forming channel 320 and the first and second culture channels 410 and 420 by injecting a cell culture medium into the sink and source channels. Long-term cell culture and blood vessel formation are possible by inducing paracrine interactions and nutrient factor supply between respective channels. In case where the blood vessel forming cells are need to be attached on the side walls of the ECM filled within the blood vessel forming channel, the blood vessel forming cells suspended in the cell culture medium can be injected into the source or the sink channel to let them attached on the desired location by tilting the blood vessel forming device by 90 degrees for 10∼15 minutes.

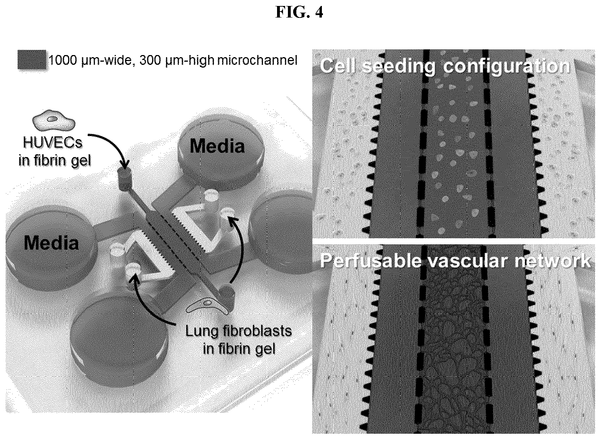

FIG. 4 illustrates the experimental configuration for the mimicry of vasculogenic blood vessel formation according to an embodiment of the present invention by injecting a mixture, which was obtained by mixing endothelial cells, HUVECs, or fibroblasts, normal human lung fibroblasts (NHLFs), with fibrin gels, into each of the blood vessel-forming channel 320 and the first and second culture channels 410 and 420; injecting a cell culture medium, endothelial cell growth medium-2 (EGM-2), into the sink channel 110 and the source channel 120. Depending on the culturing cell types and the purpose of experiments, angiogenic factors or other factors affecting cellular responses may be mixed with fibrin gels or EGM-2 to promote or modulate the cell culture and morphogenesis of blood vessel forming cells.

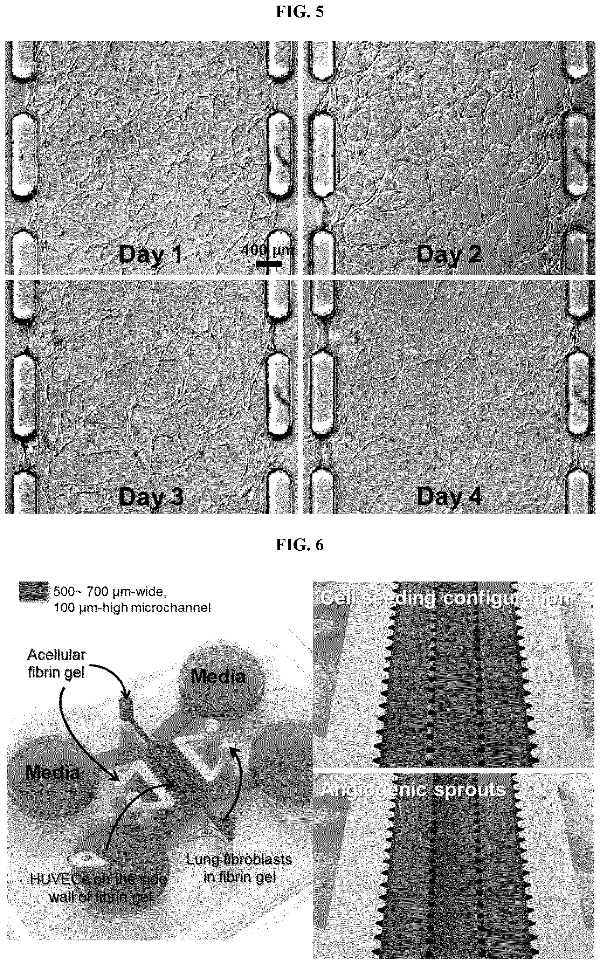

FIG. 5 is a microscopic image of a blood vessel network formed through the vasculogenesis process according to an embodiment of the present invention as illustrated in FIG. 4. As seen in the day by day microscopic observations, the blood vessel networks spontaneously emerged within the blood vessel forming channel from HUVECs embedded in fibrin matrix, resulting in complexly interconnected pattern. The blood vessels extend through the gaps formed between the microstructures 450, forming perfusable blood vessel lumens to be in fluidic communication with the cell culture medium in the sink channel 110 and the source channel 120. Therefore, it can be seen that the interior of the blood vessels is creating direct fluidic connection with cell culture medium included in the source and the sink channel, thereby the vessel lumens are directly accessible and perfusable blood vessel networks.

The present invention also provides a method of generating a blood vessel comprising a) injecting an ECM into the blood vessel-forming channel of the blood vessel-generating device; b) injecting a blood vessel-forming cell into the sink channel and/or the source channel of the blood vessel-generating device, and attaching the blood vessel-forming cell to the ECM exposed between the microstructures arranged in the sides of the blood vessel-forming channel; c) injecting i) the ECM or ii) the ECM and a co-culturing cell into the first culture channel and/or the second culture channel of the blood vessel-generating device; and d) injecting an angiogenesis factor and/or a cell culture medium into the sink channel and/or the source channel of the blood vessel-generating device and culturing the blood vessel-forming cell and the co-culturing cell, and e) interacting the blood vessel-forming cell with the co-culturing cell via paracrine signaling.

By the present method, an angiogenesis process can be simulated. To provide the directional gradient of pro-angiogenic factors to guide angiogenic sprout formation of blood vessel forming cells, the location of blood vessel forming cell attachment, injection of co-culturing cells and introduction of angiogenic factors into the source or the sink channel may be easily controlled by the one who perform the experiment.

Fig 6. Illustrates the experimental configuration for the mimicry of angiogenic blood vessel formation. Here, the angiogenic sprouts are induced by filling fibrin gels into the blood vessel-forming channel 320 to form a blood vessel-generating region 335; injecting endothelial cells, HUVECs, into the sink channel 110 disposed in the left side of the blood vessel-forming channel 320 and attaching the HUVECs to fibrin gels exposed through the gaps between the microstructures 450; injecting fibroblasts, NHLFs, and fibrin gels in the second culture channel 420 disposed in the right side of the blood vessel-forming channel 320;and injecting a cell culture medium, endothelial cell growth medium-2 (EGM-2), into the sink channel 110 and the source channel 120. Depending on the culturing cell types and the purpose of experiments, angiogenic factors or other factors affecting cellular responses may be mixed with fibrin gels or EGM-2 to promote or modulate the cell culture and morphogenesis of blood vessel forming cells. In this case, it was revealed that the angiogenic sprouts grew from the left side of the blood vessel-generating region 335 to which the HUVECs are attached, towards the direction of the second culture channel 420 in which the fibroblast NHLFs are cultured. And that the angiogenic sprouts formed perfusable blood vessels after traversing the blood vessel generating region 335.

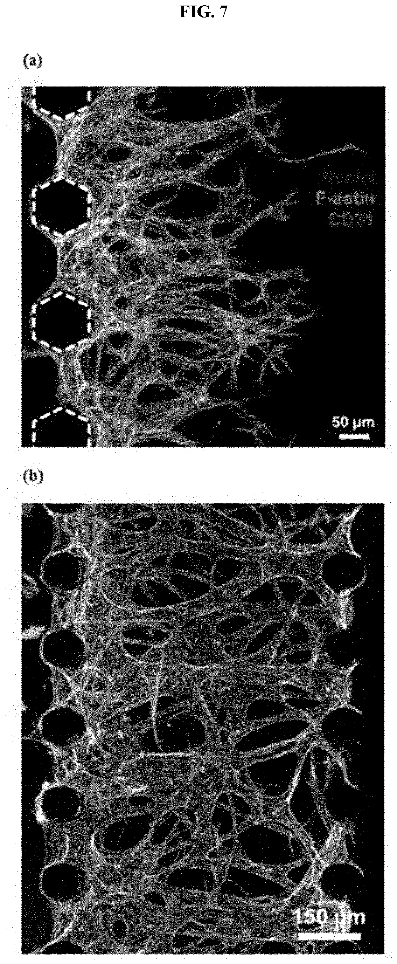

Fig. 7 (a) depicts a microscopic image of angiogenic sprouts grown for 48 hours by simulating an angiogenesis process using the device and method for generating a blood vessel according to the present invention. Fig 7 (b) depicts a picture of another blood vessel network generated by simulating an angiogenesis process 4 days after growth using the device and method for generating a blood vessel according to the present invention.

Described herein is also a blood vessel-generating device comprising a) a first culture channel in fluid communication with a first culture channel inlet, b) a blood vessel-forming channel in fluid communication with a blood vessel-forming channel inlet, disposed in contact with the sides of the first culture channel substantially parallel to the first culture channel, c) a sink channel in fluid communication with a sink channel inlet, disposed in contact with the other side of the first culture channel, substantially parallel to the first culture channel and d) a source channel in fluid communication with a source channel inlet, disposed in contact with the other side of the blood vessel-forming channel, substantially parallel to the first culture channel, wherein a plurality of microstructures configured to allow interaction between biochemical materials included in each channel are arranged in the interfaces of the respective adjacent channels at set intervals.

The present invention also provides a method of generating a blood vessel comprising
a) injecting an ECM into the blood vessel-forming channel of the blood vessel-generating device, b) injecting a blood vessel-forming cell into the source channel of the blood vessel-generating device, and attaching the blood vessel-forming cell to the ECM exposed between the microstructures arranged in the sides of the blood vessel-forming channel, c) injecting i) the ECM or ii) the ECM and a co-culturing cell into the first culture channel of the blood vessel-generating device and d) injecting an angiogenesis factor and/or a cell culture medium into the sink channel and/or the source channel of the blood vessel-generating device, and culturing the blood vessel-forming cell and the co-culturing cell, and e) interacting the blood vessel-forming cell with the co-culturing cell via paracrine signaling.

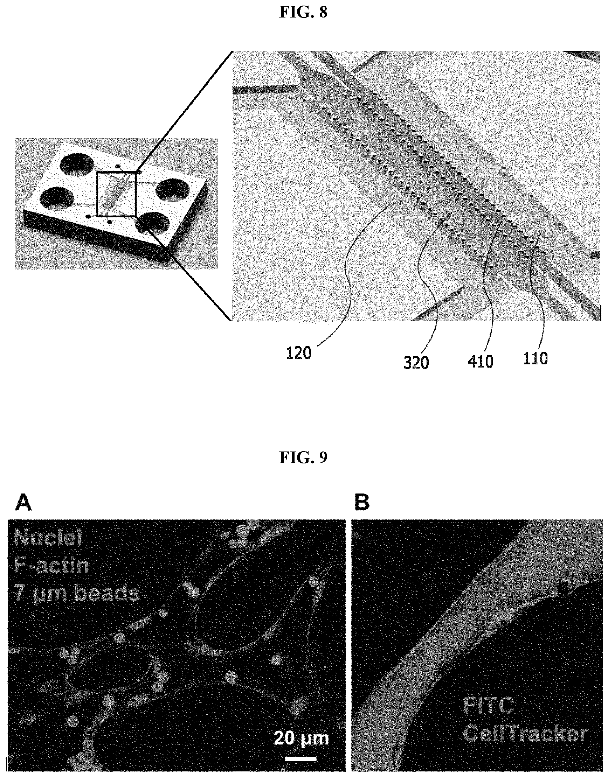

In Fig. 8, the channel 320 represents a blood vessel-forming channel, the channel 410 represents a channel injected with co-culturing cell and the channel120 or the channel 110 represents a channel injected with a cell culture medium and/or various angiogenesis factors. Fig. 8 is a view of the blood vessel generating device that carries the first culture channel as aforementioned directly in junction with the right side of the blood vessel forming channel. The process of mimicking angiogenesis in this blood vessel generating device is similar with the process described in Fig. 6. Since the blood vessel forming channels and the first culture channel, where the co-culturing cells are 3-dimensionally embedded in ECM, are directly communicated, this device has the advantage of offering a more convenient settings when the experiments are aimed to observe and modulate angiogenic sprout formation and directional growth toward pro-angiogenic factors.

The blood vessel network that is perfused and directly accessible through the source channel and the sink channel. The present invention also provides a method of perfusing a fluid into the generated vessels that are made according to angiogenesis or vasculogenesis wherein the vessels are accessible through the source channel and/or the sink channel, thereby allowing the introduction of fluid flow into the vessels. The fluid may contain at least one selected from the group consisting of a soluble factor, an insoluble factor, a biochemical material, a biomechanical stimulus and a cell. The cell contained in the fluid may be at least one selected from the group consisting of a cancer cell, an immune cell, a stem cell, a stem cell derived cell, a blood cell and a endothelial progenitor cell.

In one specific embodiment of the present invention as described above, the blood vessel-generating device 400 according to the present invention may form a concentration gradient of various signaling materials secreted by the co-culturing cells by adjusting a relative density or by culturing different kinds of cells between the co-culturing cells cultured in the first and second culture channels 410 and 420, disposed respectively on the left and right of the blood vessel-forming channel 320. In addition, the amounts of various signaling materials secreted by the co-culturing cells may be controlled by adjusting a relative density between the co-culturing cells and the blood vessel-forming cells. Also, blood vessel-generating device 400 according to the present invention may also form a concentration gradient of various angiogenesis factors and nutrient ingredients by adjusting a relative flow rate or the kind and concentration of the biochemical materials between the sink channel 110 and the source channel 120, disposed respectively on the left and right sides of the blood vessel-forming channel 320.

According to another specific embodiment of the present invention, blood vessels may be induced to have blood vessel characteristics which appear in specific organs and tissues of the human body, and specific physiological and pathological conditions of the human body by adjusting the origins and kinds of the blood vessel-forming cell, the co-culturing cell and the biochemical factors added to ECM or cell culture medium.

A fluid injected with a cell culture medium and/or an ECM may contain at least one selected from the group consisting of soluble and insoluble factors, biochemical materials, biomechanical stimuli and cells. For example, when cancer cells are injected with the blood vessel endothelial cells, the permeability of the blood vessel network may be increased in response to the various angiogenic factors and inflammation-inducing factors secreted from the cancer cells, as observed in the tumor microenvironments. On the contrary, when astrocytes are injected as the co-culturing cell, the permeability of the blood vessel network may be lowered by enhancing the tight cell-cell junctions between the cells constituting blood vessels. Also, a blood vessel network may be endowed with characteristics similar to those of the blood vessels in the brain by co-culturing neuroglial cells in an adjacent external portion of the generated blood vessel. Also, various interactions of the blood vessel-pericyte may be induced by co-culturing a mesenchymal stem cell or a pericyte, thereby realizing a cellular microenvironment in which a variety of research may be performed. In these cases, above specific cell types may be mixed in ECM to be injected into the blood vessel forming channels to support contact-dependent physical interactions with blood vessel forming cells as the blood vessels form through vasculogenes is and angiogenesis process, or may be injected into the first or the second culture channel to support contact-independent and diffusion-dependent interactions with blood vessel forming cells as described previously.

When adjusting permeability of the blood vessel network, the permeability of blood vessels may be measured by introducing a fluorescent marker and quantifying fluorescent intensity of the dye that diffuses across the vessel walls. Also, the permeability may be measured by embedding an electrode at the apical side of the vessel and the other electrode at the basal side of the vessel which allows quantitative measurement of transendothelial electrical resistance (TEER) values across the vessel walls.

In still another specific embodiment, an *in vitro* model may be provided, which may be used to understand cancer growth and the metastasis mechanism by inducing the formation of a blood vessel by a cancer cell, or disposing a cancer cell in or out of a blood vessel. In order for an angiogenesis factor secreted from a cancer cell to simulate a process of inducing morphogenesis or directional growth of blood vessel endothelial cells, the cancer cell may be mixed with an ECM to be introduced into the first and second culture channels. Also, extravasation or intravasation of cancer cells through the vessel walls can be modeled by introducing cancer cells into the apical side or the basal side of the vessel. In addition, in the above specific embodiment, cells penetrable from the blood stream into a tissue or from the tissue into the blood stream through a blood vessel wall are not limited to cancer cells. Representative examples of other cell groups that transmigrate between the tissue and the blood stream through the blood vessel wall include immune cells such as macrophages, neutrophils, pericytes, stem cells or endothelial progenitor cells. According to yet another specific embodiment, an interaction between an immune cell and a blood vessel may be simulated by introducing a fluid suspended with these immune cells into the blood vessels.

As described above, the blood vessel, which is formed in the blood vessel-generating region 335 using the blood vessel-generating device 400 according to the present invention, may communicate with the sink channel 110 and the source channel 120 through the gaps formed between the plurality of microstructures 450, as shown in FIG. 5. and FIG. 7. The images in FIG. 5 and FIG.7 illustrate the interconnection between the source and the sink channel is made through the lumen of blood vessel networks in the blood vessel forming channels, formed by mimicking both vasculogenesis and angiogenesis. In this case, various biochemical and biophysical stimulations and signals may be directly delivered into the blood vessels and the response of blood vessels can be monitored and quantified in real time.

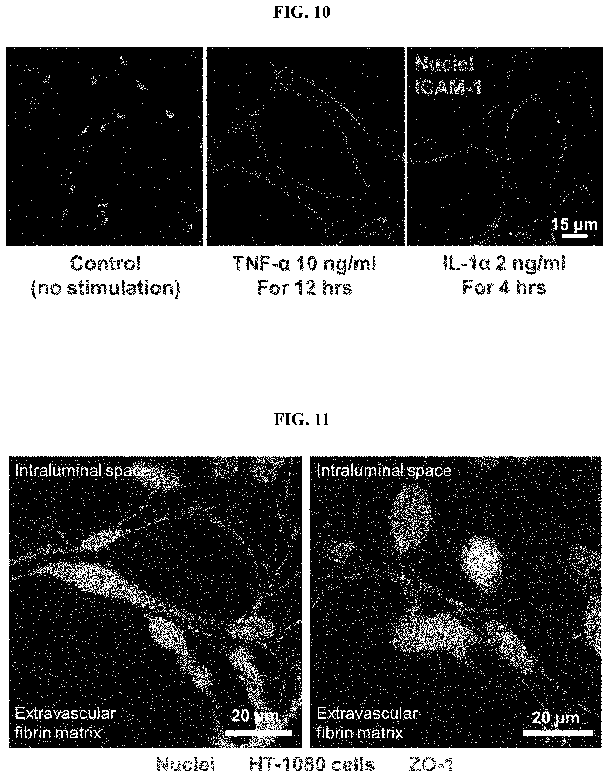

According to one specific embodiment of the present invention, a hydrostatic pressure between the sink channel 110 and the source channel 120 may be induced by adjusting the volume of the cell culture medium injected into the sink inlet 105 and/or outlet 141,and the source inlet 115 and/or outlet 142. The difference in the induced hydrostatic pressure enables the cell culture medium to flow into the blood vessel network in communication with the sink channel 110 and the source channel 120 through the gaps formed between the microstructures 450. In this case, morphological and functional characteristics such as the diameter, length, permeability, barrier function, gene and protein expression of the blood vessel may be adjusted and quantified by mixing the injected fluid with various biochemical materials that may control the characteristics of the blood vessel. Also, a flow rate of the biochemical materials flowing through the blood vessel may be controlled according to the quantitative difference in hydrostatic pressure. Therefore, the response or remodeling of a 3-dimensional blood vessel under a biophysical stimuli induced by a shear stress acting on vessel walls may be imaged and quantified. Fig 9. is a fluorescent microscopic image which shows that soluble/insoluble factors, fluorescent markers can be injected into the blood vessels connected to the source channel or the sink channel, wherein the fluorescent particles having radii of 7 micrometers and FITC (Fluorescein Isothiocyanate) solution of 70 kDa can be found. FIG. 10 is a fluorescent image that describes the inducement of ICAM-1 expression on the inside walls of the blood vessels by injecting TNF-a/IL-1a, cytokines which cause inflammation within the vessels. It shows that the fluorescent reaction was detected within the tube with the stimulation of TNF-a/IL-1a, as opposed to the control group. Furthermore, perfusing is induced and controlled by mechanical means. To maintain constant level of fluid flow through the blood vessels, syringe pumps and fluid-delivering tubes can be connected to the blood vessel generating device to monitor long term-response of blood vessels and their remodeling under biophysical stimulations. Also, applying various levels of flow into blood vessels and blood vessel network using a hydrostatic pressure or an external pump allows simulation of diverse fluid shear conditions observed in physiological and pathological situations. In addition, the perfusable blood vessel networks in 3D ECM environment can serve as a novel *in vitro* model for the simulation of endothelium and blood-borne cell interactions observed in diverse physiological and pathological phenomena. FIG. 11 is a fluorescent image which illustrates the traits of cancer cells which were introduced into the blood vessel network. The cells that have migrated across the endothelium showed stretched morphology with longer projections and sometimes fragmented cytoplasm. An extravasated cancer cell located close to the blood vessel contrast in its morphology with round cell that is still inside the lumen.

Cells had been introduced into the blood stream through culture medium in which malignant ones had been blended, whereby the cancerous cells adhere to the inside walls of the blood vessels, which leads to the penetration of the tissue.

The term 'substantially parallel' means that the channels on the plane do not intersect with each other and maintain a distance that is the same and/or relatively similar to each other.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (*e.g*., bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g*., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g*., " a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description or claims, should be understood to contemplate the possibilities of including one of the terms, either of the terms, or both terms. For example, the phrase "A or B" will be understood to include the possibilities of "A" or "B" or "A and B."

## Claims

1. A blood vessel-generating device comprising:
a) a sink channel in fluid communication with a sink inlet;
b) a source channel in fluid communication with a source inlet substantially parallel to the sink channel;
c) a blood vessel-forming channel in fluid communication with a blood vessel-forming channel inlet, disposed in contact with the sides of the sink channel and the source channel between the sink channel and the source channel, substantially parallel to the source channel and the sink channel;
d) a first culture channel in fluid communication with a first culture channel inlet, disposed in contact with the other side of the sink channel, substantially parallel to the sink channel; and
e) a second culture channel in fluid communication with a second culture channel inlet, disposed in contact with the other side of the source channel, substantially parallel to the source channel,
wherein a plurality of microstructures configured to allow interaction between biochemical materials included in each channel are arranged in the interfaces of the respective adjacent channels at set intervals, and
wherein the sink channel is disposed between the blood vessel-forming channel and the first culture channel, and the source channel is disposed between the blood vessel-forming channel and the second culture channel.

2. A method of generating a blood vessel comprising:
a) injecting an extracellular matrix (ECM) and a blood vessel-forming cell into the blood vessel-forming channel of the blood vessel-generating device according to claim 1;
b) injecting i) the ECM or ii) the ECM and a co-culturing cell into the first culture channel and/or the second culture channel of the blood vessel-generating device;
c) injecting an angiogenesis factor and/or a cell culture medium into the sink channel and/or the source channel of the blood vessel-generating device, and culturing the blood vessel-forming cell and the co-culturing cell; and
d) interacting the blood vessel-forming cell with the co-culturing cell via paracrine signaling.

3. A method of generating a blood vessel comprising:
a) injecting an extracellular matrix (ECM) into the blood vessel-forming channel of the blood vessel-generating device according to claim 1;
b) injecting a blood vessel-forming cell into the sink channel and/or the source channel of the blood vessel-generating device, and attaching the blood vessel-forming cell to the ECM exposed between the microstructures arranged in the sides of the blood vessel-forming channel;
c) injecting i) the ECM or ii) the ECM and a co-culturing cell into the first culture channel and/or the second culture channel of the blood vessel-generating device; and
d) injecting an angiogenesis factor and/or a cell culture medium into the sink channel and/or the source channel of the blood vessel-generating device, and culturing the blood vessel-forming cell and the co-culturing cell, and
e) interacting the blood vessel-forming cell with the co-culturing cell via paracrine signaling.

4. The method according to claim 2, wherein the blood vessel-forming cell is at least one selected from the group consisting of an endothelial cell, an epithelial cell, a cancer cell, a stem cell, a stem cell-derived cell, and a endothelial progenitor cell.

5. The method according to claim 4, wherein the blood vessel-forming cell is at least one selected from the group consisting of a mutated cell, a genetically modified cell and a transfected cell.

6. The method according to any of claims 2 to 5, wherein the ECM is at least one selected from the group consisting of a collagen gel, a fibrin gel, Matrigel, a self-assembled peptide gel, a polyethylene glycol gel and an alginate gel.

7. The method according to any of claims 2 to 6, wherein the co-culturing cell is at least one selected from the group consisting of an astrocyte, a glial cell, a mesothelial cell, a fibroblast, a smooth muscle cell, a cancer cell, a pericyte, a neuroglial cell, a stem cell, a stem-cell derived cell and a cell that interact with endothelium.

8. The method according to claim 7, wherein the co-culturing cell is at least one selected from the group consisting of a mutated cell, a genetically modified cell and a transfected cell.

9. The method according to any of claims 2 to 7, wherein the ECM or the cell culture medium contains at least one selected from the group consisting of a drug compound, a soluble factor, an insoluble factor, a biomolecule, a protein, a nanomaterial and siRNA.

10. The method according to claim 2 or 3, further comprising perfusing a fluid into the generated vessels, wherein the vessels are accessible through the source channel and/or the sink channel, thereby allowing the introduction of fluid flow into the vessels.

11. The method according to claim 10, wherein perfusing is induced and controlled by mechanical means.

12. The method according to claim 10, wherein the fluid contains at least one selected from the group consisting of a soluble factor, an insoluble factor, a biochemical material, a biomechanical stimulus and a cell.

13. The method according to claim 12, wherein the cell contained in the fluid is at least one selected from the group consisting of a cancer cell, an immune cell, a stem cell, a stem cell derived cell, a blood cell and an endothelial progenitor cell.

14. The method according to any of claims 2, 3 or 10, further comprising injecting a biochemical material into the generated vessels, thereby quantifying vessel barrier function of the generated blood vessel or quantifying permeability of the blood vessel.

15. The method of claim 14, wherein permeability of the blood vessel is quantified, wherein the quantifying is carried out by measuring transendothelial electrical resistance (TEER) values.

## Patentansprüche

1. Blutgefäßerzeugende Vorrichtung, umfassend:
a) einen Ausgusskanal in Fluidkommunikation mit einem Ausgusseinlass;
b) einen Quellkanal in Fluidkommunikation mit einem Quelleinlass, im Wesentlichen parallel zu dem Ausgusskanal;
c) einen blutgefäßbildenden Kanal in Fluidkommunikation mit einem blutgefäßbildenden Kanaleinlass, angeordnet in Kontakt mit den Seiten des Ausgusskanals und des Quellkanals zwischen dem Ausgusskanal und dem Quellkanal, im Wesentlichen parallel zu dem Quellkanal und dem Ausgusskanal;
d) einen ersten Kulturkanal in Fluidkommunikation mit einem ersten Kulturkanaleinlass, angeordnet in Kontakt mit der anderen Seite des Ausgusskanals, im Wesentlichen parallel zu dem Ausgusskanal, und
e) einen zweiten Kulturkanal in Fluidkommunikation mit einem zweiten Kulturkanaleinlass, angeordnet in Kontakt mit der anderen Seite des Quellkanals, im Wesentlichen parallel zu dem Quellkanal,
wobei eine Vielzahl von Mikrostrukturen, konfiguriert, um eine Interaktion zwischen biochemischen Materialien enthaltend in jedem Kanal zu ermöglichen, an den Schnittstellen der jeweiligen angrenzenden Kanäle in festgelegten Intervallen angeordnet sind, und wobei der Ausgusskanal zwischen dem blutgefäßbildenden Kanal und dem ersten Kulturkanal angeordnet ist, und der Quellkanal zwischen dem blutgefäßbildenden Kanal und dem zweiten Kulturkanal angeordnet ist.

2. Verfahren zum Erzeugen eines Blutgefäßes, umfassend:
a) Injizieren einer extrazellulären Matrix (EZM) und einer blutgefäßbildenden Zelle in den blutgefäßbildenden Kanal der blutgefäßerzeugenden Vorrichtung nach Anspruch 1;
b) Injizieren i) der EZM oder ii) der EZM und einer Co-Kultivierungszelle in den ersten Kulturkanal und/oder den zweiten Kulturkanal der blutgefäßerzeugenden Vorrichtung;
c) Injizieren eines Angiogenese-Faktors und/oder eines Zellkulturmediums in den Ausgusskanal und/oder den Quellkanal der blutgefäßerzeugenden Vorrichtung und Kultivieren der blutgefäßbildenden Zelle und der Co-Kultivierungszelle, und
d) Interagieren der blutgefäßbildenden Zelle mit der Co-Kultivierungszelle über parakrine Signalisierung.

3. Verfahren zum Erzeugen eines Blutgefäßes, umfassend:
a) Injizieren einer extrazellulären Matrix (EZM) in den blutgefäßbildenden Kanal der blutgefäßerzeugenden Vorrichtung nach Anspruch 1;
b) Injizieren einer blutgefäßbildenden Zelle in den Ausgusskanal und/oder den Quellkanal der blutgefäßerzeugenden Vorrichtung und Anheften der blutgefäßbildenden Zelle an die EZM, die zwischen den Mikrostrukturen, angeordnet in den Seiten des blutgefäßbildenden Kanals, exponiert ist;
c) Injizieren i) der EZM oder ii) der EZM und einer Co-Kultivierungszelle in den ersten Kulturkanal und/oder den zweiten Kulturkanal der blutgefäßerzeugenden Vorrichtung; und
d) Injizieren eines Angiogenese-Faktors und/oder eines Zellkulturmediums in den Ausgusskanal und/oder den Quellkanal der blutgefäßerzeugenden Vorrichtung und Kultivieren der blutgefäßbildenden Zelle und der Co-Kultivierungszelle, und
e) Interagieren der blutgefäßbildenden Zelle mit der Co-Kultivierungszelle über parakrine Signalisierung.

4. Verfahren nach Anspruch 2, wobei die blutgefäßbildende Zelle mindestens eine ist, die aus der Gruppe ausgewählt ist, die aus einer Endothelzelle, einer Epithelzelle, einer Krebszelle, einer Stammzelle, einer von einer Stammzelle abgeleiteten Zelle und einer endothelialen Vorläuferzelle besteht.

5. Verfahren nach Anspruch 4, wobei die blutgefäßbildende Zelle mindestens eine ist, die aus der Gruppe ausgewählt ist, die aus einer mutierten Zelle, einer genetisch modifizierten Zelle und einer transfizierten Zelle besteht.

6. Verfahren nach einem der Ansprüche 2 bis 5, wobei die EZM mindestens eine ist, die aus der Gruppe ausgewählt ist, die aus einem Kollagen-Gel, einem Fibrin-Gel, Matrigel, einem selbst organisierten Peptidgel, einem Polyethylenglykol-Gel und einem Alginatgel besteht.

7. Verfahren nach einem der Ansprüche 2 bis 6, wobei die Co-Kultivierungszelle mindestens eine ist, die aus der Gruppe ausgewählt ist, die aus einem Astrozyten, einer Gliazelle, einer Mesothelzelle, einem Fibroblasten, einer glatten Muskelzelle, einer Krebszelle, einem Perizyten, einer Neurogliazelle, einer Stammzelle, einer von einer Stammzelle abgeleiteten Zelle und einer Zelle, die mit einem Endothel interagiert, besteht.

8. Verfahren nach Anspruch 7, wobei die Co-Kultivierungszelle mindestens eine ist, die aus der Gruppe ausgewählt ist, die aus einer mutierten Zelle, einer genetisch modifizierten Zelle und einer transfizierten Zelle besteht.

9. Verfahren nach einem der Ansprüche 2 bis 7, wobei die EZM oder das Zellkulturmedium mindestens eines enthält, das aus der Gruppe ausgewählt ist, die aus einer Arzneimittelkomponente, einem löslichen Faktor, einem unlöslichen Faktor, einem Biomolekül, einem Protein, einem Nanomaterial und siRNA besteht.

10. Verfahren nach Anspruch 2 oder 3, weiterhin umfassend das Perfundieren eines Fluids in die erzeugten Gefäße, wobei die Gefäße durch den Quellkanal und/oder den Ausgusskanal zugänglich sind, wodurch das Einleiten eines Fluidstroms in die Gefäße möglich ist.

11. Verfahren nach Anspruch 10, wobei das Perfundieren durch mechanische Mittel erzeugt und gesteuert ist.

12. Verfahren nach Anspruch 10, wobei das Fluid mindestens eines enthält, das aus der Gruppe ausgewählt ist, die aus einem löslichen Faktor, einem unlöslichen Faktor, einem biochemischen Material, einem biomechanischen Reiz und einer Zelle besteht.

13. Verfahren nach Anspruch 12, wobei die in dem Fluid enthaltene Zelle mindestens eine ist, die aus der Gruppe ausgewählt ist, die aus einer Krebszelle, einer Immunzelle, einer Stammzelle, einer von einer Stammzelle abgeleiteten Zelle, einer Blutzelle und einer endothelialen Vorläuferzelle besteht.

14. Verfahren nach einem der Ansprüche 2, 3 oder 10, weiterhin umfassend das Injizieren eines biochemischen Materials in die erzeugten Gefäße, wodurch die Gefäßbarrierefunktion des erzeugten Blutgefäßes quantifiziert wird oder die Permeabilität des Blutgefäßes quantifiziert wird.

15. Verfahren nach Anspruch 14, wobei die Permeabilität des Blutgefäßes quantifiziert wird, wobei das Quantifizieren durch Messen der Werte des transendothelialen elektrischen Widerstandes (TEER) durchgeführt wird.

## Revendications

1. Dispositif de production de vaisseau sanguin comprenant :
a) un canal de drain en communication fluide avec une entrée de drain ;
b) un canal source en communication fluide avec une entrée de source sensiblement parallèle au canal de drain ;
c) un canal de formation de vaisseaux sanguins en communication fluide avec une entrée de canal de formation de vaisseaux sanguins, disposée en contact avec les côtés du canal de drain et du canal de source entre le canal de drain et le canal de source, sensiblement parallèle au canal de source et au canal de drain ;
d) un premier canal de culture en communication fluide avec une première entrée de canal de culture, disposée en contact avec l'autre côté du canal de drain, sensiblement parallèle au canal de drain ; et
e) un second canal de culture en communication fluide avec une seconde entrée de canal de culture, disposée en contact avec l'autre côté du canal source, sensiblement parallèle au canal source,
dans lequel une pluralité de microstructures configurées pour permettre l'interaction entre des matériaux biochimiques inclus dans chaque canal sont disposées dans les interfaces des canaux adjacents respectifs à des intervalles définis, et
dans lequel le canal de drain est disposé entre le canal de formation de vaisseau sanguin et le premier canal de culture, et le canal source est disposé entre le canal de formation de vaisseau sanguin et le second canal de culture.

2. Procédé de production d'un vaisseau sanguin comprenant :
a) l'injection d'une matrice extracellulaire (ECM) et d'une cellule formant un vaisseau sanguin dans le canal formant un vaisseau sanguin du dispositif générateur de vaisseau sanguin selon la revendication 1 ;
b) l'injection i) d'ECM ou ii) d'ECM et une cellule de co-culture dans le premier canal de culture et/ou le second canal de culture du dispositif générateur de vaisseau sanguin ;
c) injecter un facteur d'angiogenèse et/ou un milieu de culture cellulaire dans le canal de puits et/ou le canal source du dispositif de production de vaisseau sanguin, et cultiver la cellule de formation de vaisseau sanguin et la cellule de co-culture ; et
d) l'interaction entre la cellule formant le vaisseau sanguin et la cellule de co-culture par signalisation paracrine.

3. Procédé de production d'un vaisseau sanguin comprenant :
a) l'injection d'une matrice extracellulaire (ECM) dans le canal de formation de vaisseau sanguin du dispositif de production de vaisseau sanguin selon la revendication 1 ;
b) l'injection d'une cellule de formation de vaisseau sanguin dans le canal de drain et/ou le canal source du dispositif de production de vaisseau sanguin, et la fixation de la cellule de formation de vaisseau sanguin à l'ECM exposée entre les microstructures disposées sur les côtés du canal de formation de vaisseau sanguin ;
c) l'injection i) d'ECM ou ii) d'ECM et une cellule de co-culture dans le premier canal de culture et/ou le second canal de culture du dispositif de production de vaisseau sanguin ; et
d) injection d'un facteur d'angiogenèse et/ou d'un milieu de culture cellulaire dans le canal de puits et/ou le canal source du dispositif de production de vaisseau sanguin, et culture de la cellule de formation de vaisseau sanguin et de la cellule co-culture, et
e) l'interaction entre la cellule de formation de vaisseau sanguin et la cellule de co-culture par signalisation paracrine.

4. Procédé selon la revendication 2, dans lequel la cellule formant un vaisseau sanguin est au moins une cellule choisie dans le groupe constitué par une cellule endothéliale, une cellule épithéliale, une cellule cancéreuse, une cellule souche, une cellule dérivée de cellules souches et une cellule progénitrice endothéliale.

5. Procédé selon la revendication 4, dans lequel la cellule formant un vaisseau sanguin est au moins une cellule choisie dans le groupe constitué par une cellule mutée, une cellule génétiquement modifiée et une cellule transfectée.

6. Procédé selon l'une quelconque des revendications 2 à 5, dans lequel l'ECM est au moins un élément choisi dans le groupe constitué par un gel de collagène, un gel de fibrine, Matrigel, un gel peptidique auto-assemblé, un gel de polyéthylèneglycol et un gel alginate.

7. Procédé selon l'une quelconque des revendications 2 à 6, dans lequel la cellule de co-culture est au moins une cellule choisie dans le groupe constitué par un astrocyte, une cellule gliale, une cellule mésothéliale, un fibroblaste, une cellule musculaire lisse, une cellule cancéreuse, un péricycle, une cellule neurogliale, une cellule souche, une cellule dérivée de cellules souches et une cellule qui coopèrent avec l'endothélium.

8. Procédé selon la revendication 7, dans lequel la cellule de co-culture est au moins une cellule choisie dans le groupe constitué par une cellule mutée, une cellule génétiquement modifiée et une cellule transfectée.

9. Procédé selon l'une quelconque des revendications 2 à 7, dans lequel l'ECM ou le milieu de culture cellulaire contient au moins un composé choisi dans le groupe constitué par un composé médicamenteux, un facteur soluble, un facteur insoluble, une molécule biologique, une protéine, un nano matériau et un siRNA.

10. Procédé selon les revendications 2 ou 3, comprenant en outre la perfusion d'un fluide dans les vaisseaux générés, dans lequel les vaisseaux sont accessibles par le canal source et/ou le canal du puits, permettant ainsi l'introduction d'un écoulement de fluide dans les vaisseaux.

11. Procédé selon la revendication 10, dans lequel la perfusion est induite et contrôlée par des moyens mécaniques.

12. Procédé selon la revendication 10, dans lequel le fluide contient au moins un élément choisi dans le groupe constitué par un facteur soluble, un facteur insoluble, un matériau biochimique, un stimulus biomécanique et une cellule.

13. Procédé selon la revendication 12, dans lequel la cellule contenue dans le fluide est au moins une cellule choisie dans le groupe constitué par une cellule cancéreuse, une cellule immunitaire, une cellule souche, une cellule dérivée de cellules souches, une cellule sanguine et une cellule progénitrice endothéliale.

14. Procédé selon l'une quelconque des revendications 2, 3 ou 10, comprenant en outre l'injection d'un matériau biochimique dans les vaisseaux générés, quantifiant ainsi la fonction barrière du vaisseau sanguin généré ou quantifiant la perméabilité du vaisseau sanguin.

15. Procédé selon la revendication 14, dans lequel la perméabilité du vaisseau sanguin est quantifiée, la quantification étant effectuée en mesurant des valeurs de résistance électrique transendothéliale (TEER).
